# EUROPEAN PATENT APPLICATION

(11) **EP 3 949 909 A2**
(43) Date of publication of application: **09.02.2022**
(21) Application number: 20779755.6
(22) Date of filing: 11.03.2020
(51) Int. Cl.: A61F 2/46, G16H 30/40

(54) **DEVICE AND APPLICATION FOR INTEGRATED MANAGEMENT OF ARTIFICIAL JOINTS**

(30) Priority: 28.03.2019 KR 20190035492
(71) Applicant: Industry-Academic Cooperation Foundation Gyeongsang National University, Jinju-si, Gyeongsangnam-do 52828 (KR)
(72) Inventor: KANG, Yang Jae, Jinju-si Gyeongsangnam-do, 52731 (KR); YOO, Jun Il, Jinju-si, Gyeongsangnam-do 52855 (KR)
(74) Representative: Schön, Christoph
(86) International application number: PCT/KR2020/003348
(87) International publication number: WO 2020/197130

(57) **Abstract**

An artificial joint integrated management device includes an artificial joint matching unit configured to identify an artificial joint matched to a specific medical image inputted through a machine learning model and provide information on the artificial joint, and an inventory management unit configured to provide information on an inventory status of vendors for the selected artificial joints. Hence, the artificial joint integrated management device can easily provide a suitable artificial joint to a patient.

## Description

### [Technical Field]

The present disclosure relates to an artificial joint integrated management device, and more particularly to an artificial joint integrated management device and application that find an artificial joint suitable for surgery and facilitate the supply of the artificial joint.

### [Background Art]

Recently, as the history of orthopedic and surgical operations has increased, the frequency of reoperation is increasing. In particular, as more than 30 years have passed since the introduction of joint replacement surgery, the frequency of reoperations gradually increases. These surgeries are characterized by a high degree of difficulty due to a bone loss and a loss of surrounding muscles, and a poor surgical prognosis. When replacing an artificial joint in this reoperation, one of the most important things is to identify a type of artificial joint that was used before. An artificial joint applied to the hip joint consists of a combination of the acetabulum and the femur. In most patients, surgery is completed only by replacing the artificial joint corresponding to the acetabulum, but it is important to identify a type of artificial joint applied to the femur in order to determine the combination between the femur and the acetabulum. However, since all surgical records that have passed 30 years have been discarded, it is very difficult to identify the type of artificial joint, and thus a surgical method is determined by an experience of long-term instrument company employees.

In this case, there is provided a method of comparing one x-ray picture with characteristics of various artificial joints, predicting an artificial joint to be used in surgery, and ordering and using the predicted artificial joint from the vendor.

However, in this case, there is a risk that the surgery will fail because a type of predicted artificial joint is not accurate. In addition, an inventory shortage of the predicted artificial joint causes the use of suboptimal artificial joint or delay in surgery.

### [Disclosure]

### [Technical Problem]

One aspect of the present disclosure provides an artificial joint integrated management device and application capable of accurately finding suitable artificial joints required during surgery.

Another aspect of the present disclosure provides an artificial joint integrated management device and application capable of facilitating supply and demand of artificial joints.

### [Technical Solution]

In one aspect of the present disclosure, there is provided an artificial joint integrated management device comprising an artificial joint matching unit configured to identify an artificial joint matched to a specific medical image inputted through a machine learning model and provide information on the artificial joint; and an inventory management unit configured to provide information on an inventory status of vendors for selected artificial joints.

The artificial joint matching unit may include a machine learning unit configured to learn a relationship between a plurality of medical images and types of applicable artificial joints based on a database, wherein the database includes the plurality of medical images and data on artificial joints matched to the plurality of medical images; and an artificial joint information providing unit configured to provide information on the artificial joint suitable for the specific medical image based on the machine learning model generated by the machine learning unit.

The artificial joint integrated management device may further comprise a demand information providing unit configured to provide demand status information for the artificial joint.

The inventory management unit may include an inventory status management unit configured to provide an inventory possession status of the vendors for the artificial joint; and a demand status management unit configured to provide a demand status of the vendors for the artificial joint.

The inventory status management unit and the demand status management unit may be configured to be updated.

In another aspect of the present disclosure, there is provided an application stored in a storage medium that selects an artificial joint suitable for a specific medical image and provides an inventory status of selected artificial joints, the application comprising inputting the specific medical image; providing information on the artificial joint suitable for the specific medical image based on a machine learning model, wherein the machine learning model is a machine learned based on a plurality of medical images and data on artificial joints matched to the plurality of medical images; and providing information on an inventory status of a plurality of vendors for the provided artificial joint.

### [Advantageous Effects]

According to one aspect of the present disclosure, a suitable artificial joint can be easily provided to a patient.

According to one aspect of the present disclosure, it is easy to understand the inventory of artificial joints that can be used for surgery, and artificial joint vendors can prepare necessary artificial joints in advance by identifying an inventory status and a demand status of artificial joints.

### [Description of Drawings]

FIG. 1 schematically illustrates an artificial joint integrated management device according to an embodiment of the present disclosure.
FIG. 2 schematically illustrates an artificial joint matching unit according to an embodiment of the present disclosure.
FIGS. 3, 4 and 5 are flow charts illustrating execution of an artificial joint integrated management device according to an embodiment of the present disclosure.

### [Mode for Invention]

The detailed description and specific examples such as embodiments of the present disclosure are given merely by way of example, since various changes and modifications within the spirit and scope of the present disclosure will become apparent to those skilled in the art from the detailed description.

Wherever possible, the same reference numbers will be used throughout the drawings to refer to the same or like parts or components.

Terms used in the present disclosure are used to explain embodiments and are not intended to limit and/or restrict the present disclosure. A singular expression can include a plural expression as long as it does not have an apparently different meaning in context. In the present disclosure, terms "include" and "have" should be understood to be intended to designate that illustrated features, numbers, steps, operations, components, parts or combinations thereof are present and not to preclude the existence of one or more different features, numbers, steps, operations, components, parts or combinations thereof, or the possibility of the addition thereof.

The terms including an ordinal number such as "first", "second", etc. may be used to describe various components, but the components are not limited by such terms. The terms are used only for the purpose of distinguishing one component from other components. For example, a first component may be referred to as a second component without departing from the spirit and scope of the present disclosure, and a second component may be referred to as a first component in the same manner. The term "and/or" includes a combination of items related to plurality or some of items related to plurality.

In addition, term such as "part", "device", "block", "member", and "module" may refer to a unit processing at least one function or operation. For example, the terms may mean at least one hardware such as field-programmable gate array (FPGA)/application specific integrated circuit (ASIC), at least one software stored in a memory, or at least one process processed by a processor.

Embodiments of the present disclosure are described in detail below with reference to the accompanying drawings.

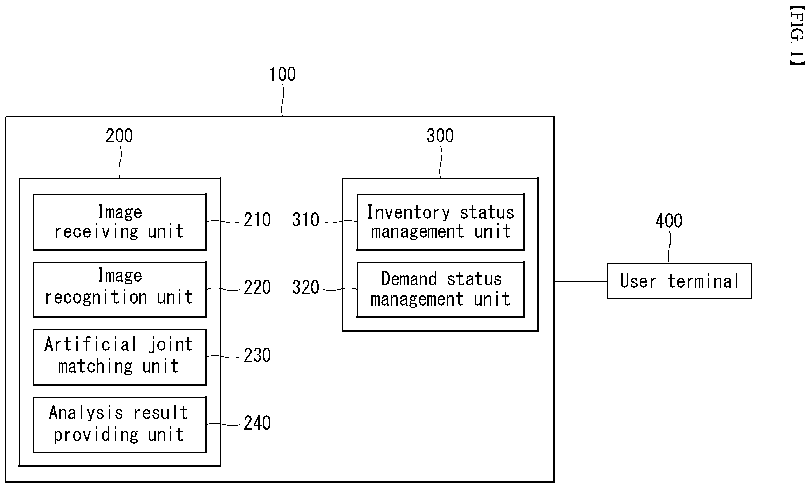

FIG. 1 schematically illustrates an artificial joint integrated management device 100 according to an embodiment of the present disclosure.

The artificial joint integrated management device 100 can accurately identify and select types of suitable artificial joints from only a medical image, and is configured to facilitate the supply of the selected artificial joints. The artificial joint integrated management device 100 can be applied to not only reoperation but also surgery performed for the first time.

The artificial joint integrated management device 100 may roughly include an artificial joint selection unit 200 and an inventory management unit 300.

The artificial joint selection unit 200 is configured to select suitable artificial joints for a specific image based on information of database and provide information on the selected artificial joints. The artificial joint selection unit 200 is described in detail later.

The inventory management unit 300 is configured to provide information on an inventory status of the selected artificial joints.

The inventory management unit 300 is configured to provide information on the inventory status and a demand status of the artificial joints selected by the artificial joint selection unit 200. The inventory management unit 300 may include an inventory status management unit 310 that stores and provides information on an inventory status for each of types of artificial joints for a plurality of vendors, and a demand status management unit 320 that stores and provides information on a demand status for each of types of artificial joints for the plurality of vendors. The plurality of vendors may access the inventory status management unit 310 and the demand status management unit 320 to update information.

A user terminal 400 is not particularly limited as long as it has a communication function connected to the integrated management device and a display function capable of outputting an image or text. Examples of the user terminal 400 may include a desktop computer, a laptop computer, a tablet PC, a wireless phone, a mobile phone, a smart phone, a smart watch, a smart glass, an e-book reader, a portable multimedia player (PMP), a portable game machine, a navigation device, a digital camera, a digital multimedia broadcasting (DMB) player, a digital audio recorder, a digital audio player, a digital picture recorder, a digital picture player, a digital video recorder, and a digital video player, but the present disclosure is not limited thereto. Herein, a user may include a doctor or a medical institution as an expert, a vendor selling artificial joints, and the like. In addition, the user may include a person, an institution, or a company with an access right to the corresponding device.

The doctor or the medical institution can grasp a possession status of artificial joints for the plurality of vendors to easily find a vendor that sells a necessary artificial joint.

The plurality of vendors may be provided to check an inventory status and a demand status of other vendors. Hence, the vendors can manage the inventory by grasping a status of artificial joints that are frequently used in practice

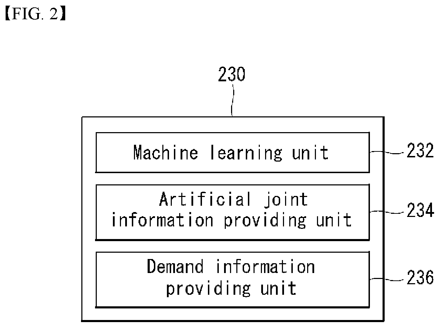

FIG. 2 schematically illustrates an artificial joint matching unit 230 according to an embodiment of the present disclosure. This is described with reference to FIG. 1 together.

The artificial joint selection unit 200 may include an image receiving unit 210, an image recognition unit 220, an artificial joint matching unit 230, and an analysis result providing unit 240.

The image receiving unit 210 may be configured to receive input specific image information, and the image recognition unit 220 may be configured to recognize a specific part required in the received specific image information. The image receiving unit 210 may include a segmentation process of detecting and displaying a target region in an input image. A method of segmenting an image may include a threshold-based segmentation, an area extension, an active contour, a level set method, a watershed, etc.

The artificial joint matching unit 230 is configured to select suitable artificial joints from an input specific medical image based on machine learning.

The artificial joint matching unit 230 may include a machine learning unit 232 and an artificial joint information providing unit 234.

The machine learning unit 232 is configured to perform machine learning by applying an algorithm based on the information stored in the database and generate a related machine learning model. The machine learning unit 232 is configured to generate a machine learning model for learning a relationship between a plurality of medical images stored in the database and the applied artificial joints.

The database may store the plurality of medical images and data on types of artificial joints that have been applied. The database may also store artificial joints that have been imported to Korea for the past 30 years and medical images to which the corresponding artificial joints have been applied.

The database may be newly updated with new medical images, information on artificial joints, information determined by the artificial joint integrated management device 100, and the like.

The medical image may include various medical images including X-ray, ultrasound, computerized tomography (CT), magnetic resonance imaging (MRI), positron emission tomography (PET), etc.

An algorithm such as logistic regression, a support vector machine, gradient boosting, and random forest may be applied to the generation of machine learning model. If the machine learning for the relationship between the medical images and the applied artificial joints is performed, a type of algorithm, that satisfies the relationship and is applied, is not limited.

The artificial joint information providing unit 234 selects artificial joints suitable for the target region through the image recognition unit 220 based on the machine learning model generated by the machine learning unit 232.

The artificial joint matching unit 230 may include a demand information providing unit 236.

The demand information providing unit 236 is configured to provide together practical demand information for the artificial joints selected by the artificial joint information providing unit 234 or a preference for each type of artificial joints. The practical demand information or the preference for each type of artificial joints that are stored in and provided by the demand information providing unit 236 may be received from the database, in which the existing demand status has been accumulated and stored, or the demand status of the inventory management unit 300.

The analysis result providing unit 240 may be configured to exchange information between the user terminal 400 and the artificial joint integrated management device 100. The analysis result providing unit 240 may transmit, to the user terminal 400, information on the artificial joint provided by the artificial joint matching unit 230, the practical demand information, or the preference for each type of artificial joints. The analysis result providing unit 240 may also transmit, to the user terminal 400, information provided by the inventory management unit 300.

The user terminal 400 may include a communication unit, a display, an input/output unit, a processor, and a storage unit.

The user may download an application for the artificial joint integrated management through the communication unit. The downloaded application may be stored in the memory.

The communication unit runs the application by the process to transmit a medical image obtained by at least one imaging device to the artificial joint integrated management device 100 and receive information on the matched artificial joint or information on an inventory status, a demand status, etc. thereof.

The display may display the information on the transmitted medical image, artificial joint, or inventory and demand statuses using at least one of an image type user interface (UI) and a text type user interface.

The processor may run the artificial joint integrated management application. The processor may select a specific medical image and select at least a part of a result received from the artificial joint integrated management device 100.

The input/output unit may output data or information about medical images and artificial joints through an input device or an output device and may obtain a user input. The input/output unit may process an image or text based user input in the user interface displayed on the display under the control of the processor.

The memory may store the specific medical image and content received from the artificial joint integrated management device 100. The memory may also store information about the artificial joint matching the specific medical image selected by the processor. The information may be transmitted to the artificial joint integrated management device 100 via the communication unit and may be stored in the database.

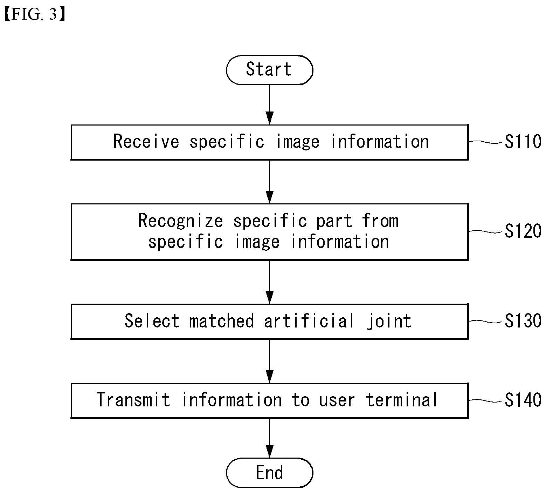

FIG. 3 is a flow chart illustrating execution of the artificial joint integrated management device according to an embodiment of the present disclosure.

The artificial joint integrated management device 100 receives specific image information from the user terminal, in S110. The artificial joint integrated management device 100 recognizes a specific part from the specific image information through the image recognition unit 220, in S120. The artificial joint integrated management device 100 selects an artificial joint suitable for the specific part recognized by the image recognition unit 220 through a machine learning model that is machine learned through existing data, in S130.

The artificial joint integrated management device 100 transmits information on the selected artificial joint to the user terminal, in S140.

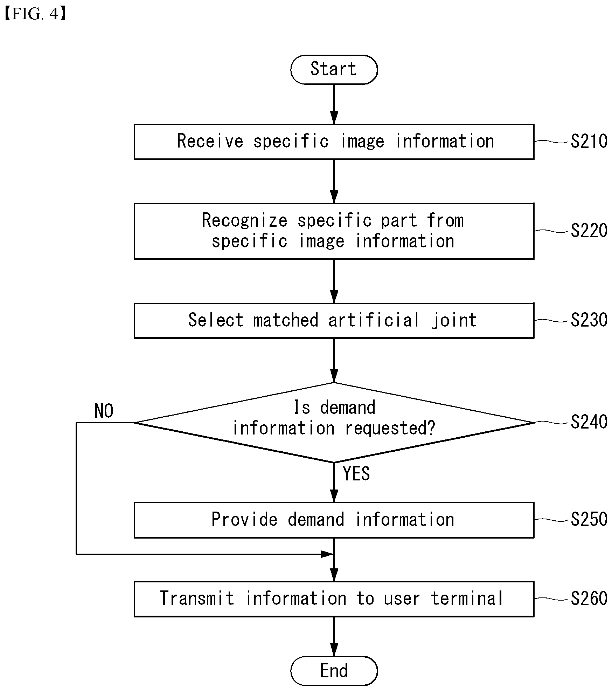

FIG. 4 is a flow chart illustrating execution of the artificial joint integrated management device 100 according to an embodiment of the present disclosure.

The artificial joint integrated management device 100 receives specific image information from the user terminal, in S210. The artificial joint integrated management device 100 recognizes a specific part from the specific image information through the image recognition unit 220, in S220. The artificial joint integrated management device 100 selects an artificial joint suitable for the specific part recognized by the image recognition unit 220 through a machine learning model that is machine learned through existing data, in S230.

Depending on whether there is a request for practical demand information of the selected artificial joint or a preference for each type of artificial joints (S240), if there is a request, the artificial joint integrated management device 100 provides the practical demand information of the selected artificial joint or information on the preference for each type of artificial joints through the demand information providing unit, in S250.

The artificial joint integrated management device 100 transmits, to the user terminal, information on the selected artificial joint, the practical demand information according to the request, or information on the preference for each type of artificial joints, in S260.

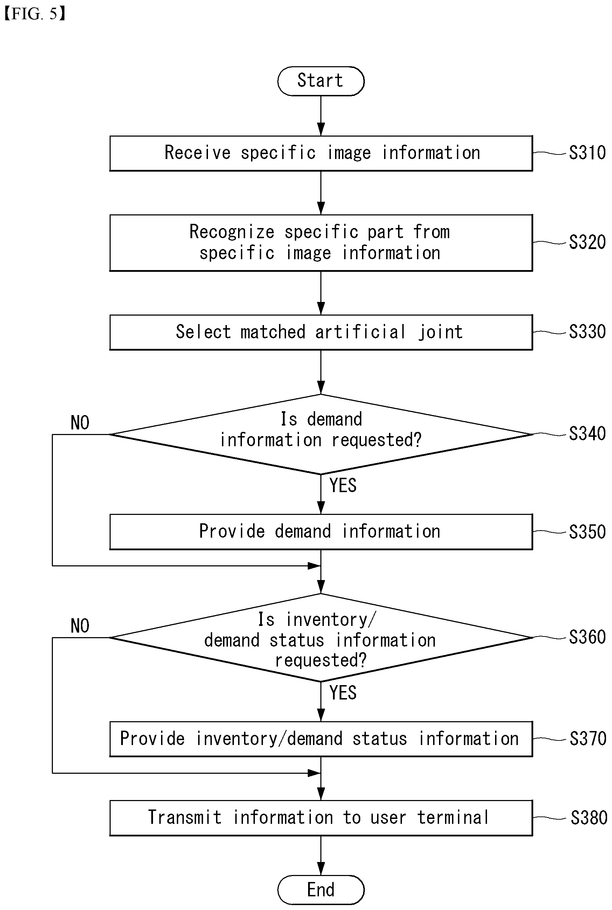

FIG. 5 is a flow chart illustrating execution of the artificial joint integrated management device 100 according to an embodiment of the present disclosure.

The artificial joint integrated management device 100 receives specific image information from the user terminal, in S310. The artificial joint integrated management device 100 recognizes a specific part from the specific image information through the image recognition unit 220, in S320. The artificial joint integrated management device 100 selects an artificial joint suitable for the specific part recognized by the image recognition unit 220 through a machine learning model that is machine learned through existing data, in S330.

Depending on whether there is a request for practical demand information of the selected artificial joint or a preference for each type of artificial joints (S340), if there is a request, the artificial joint integrated management device 100 provides the practical demand information of the selected artificial joint or information on the preference for each type of artificial joints through the demand information providing unit, in S350.

According to an information request for an inventory status and a demand status of a plurality of vendors for the selected artificial joint (S360), if there is a request, the artificial joint integrated management device 100 provides information on an inventory status and a demand status for each of the plurality of vendors for the selected artificial joint, in S370.

The artificial joint integrated management device 100 transmits, to the user terminal, information on the selected artificial joint, the practical demand information of the artificial joint according to the request, the preference for each type of artificial joints, or information on the inventory status and the demand status according to the request, in S360.

As described above, the artificial joint integrated management device 100 according to the present disclosure can accurately identify types of suitable artificial joints from limited medical images, and can understand demand information or a preference of the corresponding artificial joints based on this. Hence, consumers of artificial joints such as doctors and medical institutions can accurately identify suitable artificial joints and know their inventory status, and suppliers of artificial joints such as vendors can check an inventory status, a preference status, and a demand status and thus can supply smoothly the artificial joints.

Although the embodiments have been described with reference to a number of illustrative embodiments thereof, numerous other modifications and embodiments may be devised by those skilled in the art that will fall within the scope of the principles of the present disclosure. In particular, various variations and modifications are possible in the component parts and/or arrangements of the subject combination arrangement within the scope of the present disclosure, the drawings and the appended claims.

| | | | |
|---|---|---|---|
| 100: | artificial joint integrated management device | | |
| 200: | artificial joint selection unit | 210: | image receiving unit |
| 220: | image recognition unit | 230: | artificial joint matching unit |
| 232: | machine learning unit | 234: | artificial joint information providing unit |
| 236: | demand information providing unit | 240: | analysis result providing unit |
| 300: | inventory management unit | 400: | user terminal |

## Claims

1. An artificial joint integrated management device comprising:
an artificial joint matching unit configured to identify an artificial joint matched to a specific medical image inputted through a machine learning model and provide information on the artificial joint; and
an inventory management unit configured to provide information on an inventory status of vendors for selected artificial joints.

2. The artificial joint integrated management device of claim 1, wherein the artificial joint matching unit includes:
a machine learning unit configured to learn a relationship between a plurality of medical images and types of applicable artificial joints based on a database, wherein the database includes the plurality of medical images and data on artificial joints matched to the plurality of medical images; and
an artificial joint information providing unit configured to provide information on the artificial joint suitable for the specific medical image based on the machine learning model generated by the machine learning unit.

3. The artificial joint integrated management device of claim 2, further comprising:
a demand information providing unit configured to provide demand status information for the artificial joint.

4. The artificial joint integrated management device of claim 2, wherein the inventory management unit includes:
an inventory status management unit configured to provide an inventory possession status of the vendors for the artificial joint; and
a demand status management unit configured to provide a demand status of the vendors for the artificial joint.

5. The artificial joint integrated management device of claim 4, wherein the inventory status management unit and the demand status management unit are configured to be updated.

6. An application stored in a storage medium that selects an artificial joint suitable for a specific medical image and provides an inventory status of selected artificial joints, the application comprising:
inputting the specific medical image;
providing information on the artificial j oint suitable for the specific medical image based on a machine learning model, wherein the machine learning model is a machine learned based on a plurality of medical images and data on artificial joints matched to the plurality of medical images; and
providing information on an inventory status of a plurality of vendors for the provided artificial joint.
